# EUROPEAN PATENT SPECIFICATION

(11) **EP 3 157 328 B1**
(45) Date of publication and mention of the grant of the patent: **04.08.2021**
(21) Application number: 15735780.7
(22) Date of filing: 17.06.2015
(51) Int. Cl.: A01K 67/027, C12N 9/22, C12N 15/85

(54) **A METHOD FOR DIRECTING PROTEINS TO SPECIFIC LOCI IN THE GENOME AND USES THEREOF**
VERFAHREN ZUM LENKEN VON PROTEINEN ZU SPEZIFISCHEN LOCI IM GENOM UND VERWENDUNGEN DAVON
PROCÉDÉ POUR DIRIGER DES PROTÉINES VERS DES LOCI SPÉCIFIQUES DANS LE GÉNOME ET LEURS UTILISATIONS

(30) Priority: 17.06.2014 US 201462013382 P; 19.05.2015 US 201562163565 P
(43) Date of publication of application: 26.04.2017
(73) Proprietor: Poseida Therapeutics, Inc., San Diego, California 92121 (US)
(72) Inventor: OSTERTAG, Eric, Del Mar, CA 92014-2617 (US); YESHI, Tseten, Lexington, Kentucky 40502 (US); LI, Xianghong, San Diego, CA 92130-5515 (US)
(74) Representative: Cooley (UK) LLP
(86) International application number: PCT/US2015/036226
(87) International publication number: WO 2015/195798

(56) References cited:
- EP-A1- 2 522 726
- VANNOCCI TOMMASO ET AL: "Nuclease-stimulated homologous recombination at the human beta-globin gene", January 2014 (2014-01), JOURNAL OF GENE MEDICINE, VOL. 16, NR. 1-2, PAGE(S) 1-10, XP002743931, ISSN: 1099-498X(print) page 4, column 2, last paragraph - page 5, column 1, paragraph 1; figure 2 page 2, column 2, paragraph 2
- YI LI ET AL: "Transcription activator-like effector hybrids for conditional control and rewiring of chromosomal transgene expression", SCIENTIFIC REPORTS, vol. 2, 28 November 2012 (2012-11-28), XP055071520, DOI: 10.1038/srep00897
- KEITH JOUNG, J.K: "Dimeric CRISPR RNA-guided Fokl nucleases for highly specific genome editing", NATURE BIOTECHNOLOGY, 25 April 2014 (2014-04-25), XP55178523, cited in the application
- PRASHANT MALI ET AL: "CAS9 transcriptional activators for target specificity screening and paired nickases for cooperative genome engineering", NATURE BIOTECHNOLOGY, vol. 31, no. 9, 1 August 2013 (2013-08-01) , pages 833-838, XP055186073, ISSN: 1087-0156, DOI: 10.1038/nbt.2675
- LUKE A. GILBERT ET AL: "CRISPR-Mediated Modular RNA-Guided Regulation of Transcription in Eukaryotes", CELL, vol. 154, no. 2, 1 July 2013 (2013-07-01), pages 442-S5, XP055167546, ISSN: 0092-8674, DOI: 10.1016/j.cell.2013.06.044
- L. CONG ET AL: "Multiplex Genome Engineering Using CRISPR/Cas Systems", SCIENCE, vol. 339, no. 6121, 15 February 2013 (2013-02-15), pages 819-823, XP055181426, ISSN: 0036-8075, DOI: 10.1126/science.1231143
- VIELEMEYER OLE ET AL: "Characterization of single chain antibody targets through yeast two hybrid", BMC BIOTECHNOLOGY, BIOMED CENTRAL LTD. LONDON, GB, vol. 10, no. 1, 22 August 2010 (2010-08-22), page 59, XP021076454, ISSN: 1472-6750, DOI: 10.1186/1472-6750-10-59
- TANENBAUM MARVIN E ET AL: "A Protein-Tagging System for Signal Amplification in Gene Expression and Fluorescence Imaging", CELL, ELSEVIER, AMSTERDAM, NL, vol. 159, no. 3, 9 October 2014 (2014-10-09), pages 635-646, XP029084861, ISSN: 0092-8674, DOI: 10.1016/J.CELL.2014.09.039

## Description

### Field of the Disclosure

The present disclosure relates generally to methods for site-directed genome modification.

### Background

There are many instances in which it would be desirable to localize a protein to a specific locus in the genome of an organism in order for the protein to carry out a specific function. For example, a protein might serve the function of cutting DNA, methylating DNA, inducing fluorescence, etc. Most proteins have endogenous DNA binding domains that either target many sites in the genome (which results in poor specificity) or can only target a single site in the genome (limiting the ability to customize the targeting). It is, therefore, oftentimes desirable to remove the endogenous DNA binding domain from a protein and replace it with the DNA binding domain from another protein which has more desirable features. Alternatively, it may be desirable to add a DNA binding domain from another protein in order to localize a protein to a site that is not normally bound by that protein. This strategy has been used with great success, for example, in the gene editing field by fusing a modular DNA binding domain, such as a zinc finger array, a transcription activator-like array, or a Cas9 protein (which can be directed to a specific site in the genome through a "guide RNA", to a nuclease domain).

One instance in which it is desirable to localize a protein to a specific location in the genome is in the case of gene editing. In all such examples of gene editing tools, a DNA binding domain is fused to a nuclease domain through a covalent linkage via a peptide bond. This is easily carried out by adding the DNA coding sequence of one protein downstream of the coding sequence for a second protein, such that the two will be translated as a single polypeptide (European Patent Application EP 2 522 726). However, one problem with this strategy is that the protein can only be linked conveniently to another protein at the protein's amino terminus (N-terminus) or carboxy terminus (C-terminus). Unfortunately, attaching a protein in this manner will oftentimes cause one or both of the proteins to fold incorrectly, thereby increasing the likelihood of compromised function. Even if the fused proteins do, in fact, fold correctly, it is not uncommon for one or both of the fused proteins to be non-functional due to one protein physically blocking the ability of the other protein to function normally as a result of the covalent bond. These problems may sometimes alleviated by the use of a flexible linker that is encoded between the two polypeptides. However, many protein fusions are still not functional despite the use of a linker sequence. For example, even when an acceptable linker is found, the specific architecture may still greatly limit the function of the fusion protein. For example, it has been shown that a FokI-dCas9 fusion protein must always be in "PAM out" configuration and must contain a certain spacer region (Keith Joung, J.K, "Dimeric CRISPR RNA-guided Fok1 nucleases for highly specific genome editing," Nature Biotechnology 2014). Thus, despite the advantages of a linker, this method still greatly limits the number of sites that can be successfully targeted.

Another problem with the use of fusion protein strategies such as those described above, is that the process creates one large protein that is much larger than either of the individual single proteins. This too can compromise function or the ability of the fused protein to access the desired locations in vivo. Further, it is often desirable to instead deliver DNA that encodes for the desired fused protein into cells via viral delivery methods. However, viral delivery methods are limited by the amount of DNA that they can contain. DNA encoding large fusion proteins may not fit in viral delivery vehicles (such as, for example, Adeno Associated Virus (AAV)), thereby limiting the utility of this method.

Thus, the methods known in the art for gene editing using fusion proteins are currently limited and have one or more of the problems described above. The instant disclosure seeks to address one or more such problems in the art.

### Summary

The invention, which provides a method of modifying a genome of an organism *in vitro* or *ex vivo,* is defined by the appended claim set.

Disclosed are compositions and methods for directing proteins to specific loci in the genome and uses thereof. In one aspect, the disclosed methods allow for directing proteins to specific loci in the genome of an organism, including the steps of providing a DNA localization component and an effector molecule, wherein the DNA localization component and the effector molecule are capable of being operatively linked via a non-covalent linkage.

The disclosure provides a method for directing proteins to specific loci in a genome of an organism comprising the steps of (a) providing a DNA localization component; and (b) providing an effector molecule; wherein the DNA localization component and the effector molecule are capable of operatively linking via a non-covalent linkage. In certain embodiments of this method, the DNA localization component is capable of binding a specific DNA sequence.

The disclosure provides a method for modifying a genome of an organism comprising the steps of (a) providing a DNA localization component; and (b) providing an effector molecule; wherein the DNA localization component and the effector molecule are capable of operatively linking via a non-covalent linkage. According to this method, a genome may be modified when one or more genomic sequences or base pairs are separated by an endonuclease and/or when one or more genomic sequences or base pairs are deleted, inserted, substituted, inverted, or relocated. Moreover, the disclosure provides a cell comprising a genomic sequence or base pair modified by a method of the disclosure. Cells modified by the methods of the disclosure may comprise, for example, a deletion, an insertion, a substitution, an inversion, or a relocation of a genomic sequence or base pair of the genome. Cells modified according to the methods of the disclosure may comprise, for example, an exogenous, artificial, or heterologous sequence that does not naturally-occur within the genome of that cell. The cell may be modified according to a method of the disclosure in vivo, ex vivo, or in vitro. In certain embodiments, the cell is neither a human cell nor a human embryonic cell.

Exemplary DNA localization components of the disclosure include, but are not limited to, a DNA-binding oligonucleotide, a DNA-binding protein, a DNA binding protein complex, and any combination thereof.

DNA localization components of the disclosure may comprise an oligonucleotide directed to a specific locus in the genome. Exemplary oligonucleotides include, but are not limited to, DNA, RNA, DNA/RNA hybrids, and any combination thereof.

DNA localization components of the disclosure may comprise a protein or a protein complex capable of recognizing a feature selected from RNA-DNA heteroduplexes, R-loops, and any combination thereof. Exemplary proteins or protein complexes capable of recognizing an R-loop include, but are not limited to, Cas9, Cascade complex, RecA, RNase H, RNA polymerase, DNA polymerase, and any combination thereof. In certain embodiments of the methods of the disclosure, the protein or protein complex capable of recognizing an R-loop comprises Cas9.

DNA localization components of the disclosure may comprise a protein capable of binding a DNA sequence selected from meganuclease, Zinc Finger array, TAL array, and any combination thereof.

DNA localization components of the disclosure may comprise a protein comprising a naturally occurring DNA binding domain. Exemplary naturally occurring DNA binding domains include, but are not limited to, a bZIP domain, a Helix-loop-helix, a Helix-turn-helix, a HMG-box, a Leucine zipper, a Zinc finger, and any combination thereof.

DNA localization components of the disclosure may comprise an oligonucleotide directed to a target location in a genome and a protein capable of binding to a target DNA sequence.

Exemplary effector molecules of the disclosure are capable of a predetermined effect at a specific locus in the genome.

Exemplary effector molecules of the disclosure include, but are not limited to, a transcription factor (activator or repressor), chromatin remodeling factor, nuclease, exonuclease, endonuclease, transposase, methytransferase, demethylase, acetyltransferase, deacetylase, kinase, phosphatase, integrase, recombinase, ligase, topoisomerase, gyrase, helicase, fluorophore, or any combination thereof.

Exemplary effector molecules of the disclosure comprise a nuclease. Non-limiting examples of nucleases include restriction endonucleases, homing endonucleases, S1 Nuclease, mung bean nuclease, pancreatic DNase I, micrococcal nuclease, yeast HO endonuclease, or any combination thereof. In certain embodiments, the effector molecule comprises a restriction endonuclease. In certain embodiments, the effector molecule comprises a Type IIS restriction endonuclease.

Exemplary effector molecules of the disclosure may comprise an endonuclease. Non-limiting examples of the endonuclease include AciI, Mn1I, AlwI, BbvI, BccI, BceAI, BsmAI, BsmFI, BspCNI, BsrI, BtsCI, HgaI, HphI, HpyAV, MbolI, MylI, PleI, SfaNI, AcuI, BciVI, BfuAI, BmgBI, Bmrl, BpmI, BpuEI, BsaI, BseRI, BsgI, BsmI, BspMI, BsrBI, BsrBI, BsrDI, BtgZI, BtsI, Earl, EciI, MmeI, NmeAIII, BbvCI, Bpu10I, BspQI, SapI, BaeI, BsaXI, CspCI, FokI, BfiI, MboII, Acc36I and Clo051. In certain embodiments, the effector molecule comprises Bmrl, BfiI, or Clo051. The effector molecule may comprise BmrI. The effector molecule may comprise BfiI. The effector molecule may comprise Clo051.The effector molecule may comprise FokI.

Exemplary effector molecules of the disclosure may comprise a transposase.

Exemplary non-covalent linkages of the disclosure may comprise an antibody fragment covalently attached to an effector molecule, which non-covalently binds directly to a DNA localization component.

Exemplary non-covalent linkages of the disclosure may comprise an antibody fragment covalently attached to a DNA localization component, non-covalently binds directly to an effector component.

Exemplary non-covalent linkages of the disclosure may comprise an antibody fragment covalently attached to either an effector molecule or a DNA localization component, which non-covalently binds to an epitope tag covalently attached to the opposite component. In certain embodiments of the disclosure, antibody fragments may comprise or consist of a single-chain variable fragment (scFv), a single domain antibody (sdAB), a small modular immunopharmaceutical (SMIP) molecule, or a nanobody.

Exemplary non-covalent linkages of the disclosure may comprise a protein binding domain covalently attached to either an effector molecule or a DNA localization component, which non-covalently binds to the opposite component

Exemplary non-covalent linkages of the disclosure may comprise a protein covalently attached to either an effector molecule or a DNA localization component capable of binding to a protein covalently attached to the opposite component.

Non-covalent linkages of the disclosure may comprise or consist of an antibody mimetic. Exemplary antibody mimetics include, but are not limited to, an organic compound that specifically binds a target sequence and has a structure distinct from a naturally-occurring antibody. Moreover, Exemplary antibody mimetics include, but are not limited to, a protein, a nucleic acid, or a small molecule. In certain embodiments of the disclosure, the antibody mimetic comprises or consists of an affibody, an afflilin, an affimer, an affitin, an alphabody, an anticalin, and avimer, a DARPin, a Fynomer, a Kunitz domain peptide, or a monobody.

Exemplary non-covalent linkages of the disclosure may comprise a small molecule covalently attached either to an effector molecule or a DNA localization component, which non-covalently binds to a protein or other small molecule covalently attached to the opposite component.

### Brief Description of the Drawings

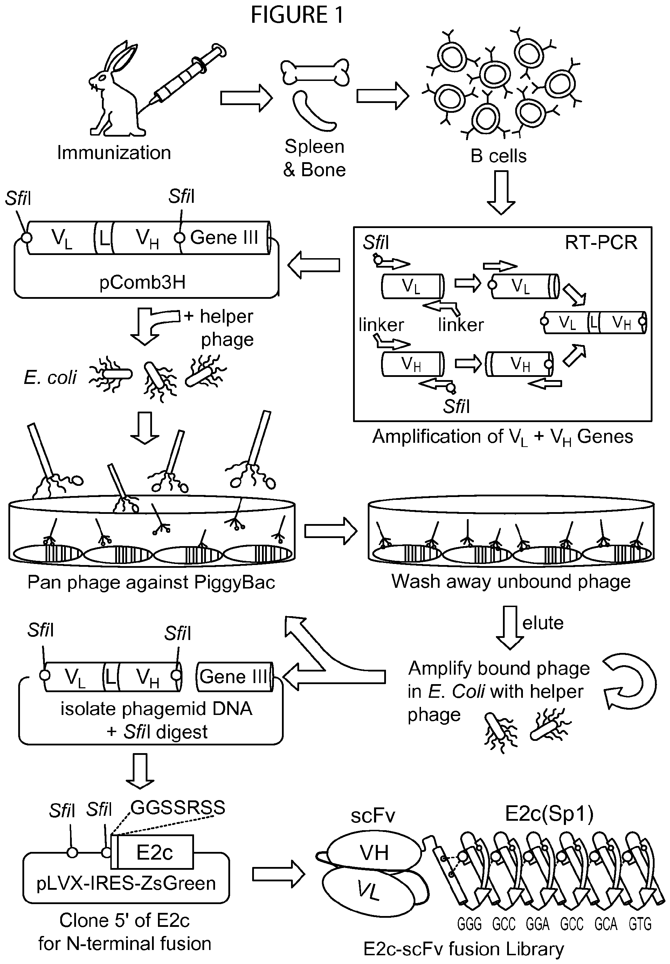

Figure 1 is a schematic representation depicting the method of phage display to generate scFv against piggyBac. Rabbits are immunized with PB transposase protein (PBase) for expanding relevant B cells. Variable regions from heavy and light chain (VH and VL) genes are amplified from cDNA by PCR to form fusion products containing an 18 amino acid linker (L). Phagemid are produced, panned against PBase, amplified in E.coli, and repeated once or twice. The resulting phagemid DNA library is cloned into the pLVX-IRES-ZsGreen vector containing the E2c PZF with a linker sequence. An E2c-scFv N-terminal fusion library is then produced in Lentivirus.

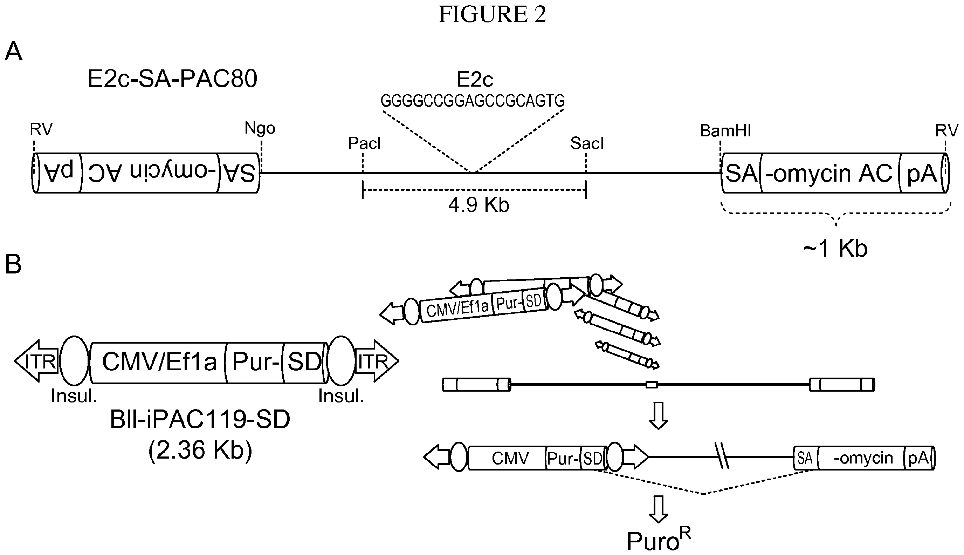

Figures 2A and 2B are a pair of schematic representations depicting site-specific complementation. Figure 2A shows that the E2c-SA-PAC141 cassette contains an E2c site (GGGGCCGGAGCCGCAGTG; SEQ ID NO: 1) located in the center of a 10.47 Kb *NgoMIV-Bam*HI fragment from the p53 intron 1, flanked by 2 inverted copies of the Adenovirus 2 splice acceptor (SA), the 141 amino acid C-terminal fragment of the puromycin acetyltransferase (PAC) gene followed by an SV40 polyadenylation (pA) signal. Figure 2B shows that the BII-iPAC58-SD transposon contains a CpG-less promoter consisting of the cytomegalovirus (CMV) enhancer and a human *Ef1a* promoter that drives expression of the N-terminal fragment of the PAC gene. An Ad2 splice donor provides a poly-A trap. Insulators (Insul.) from the chicken beta globin locus HSIV ensures stable expression. Insertions upstream of the E2c-SA-PAC141 cassette result in splicing and production of a functional PAC transcript.

### Detailed Description

### Definitions

The present disclosure may be understood more readily by reference to the following detailed description of preferred embodiments of the disclosure and the Examples included therein and to the Figures and their previous and following description. Although any methods and materials similar or equivalent to those described herein can be used in the practice or testing of the present invention, the preferred methods, devices, and materials are now described. All references, publications, patents, patent applications, and commercial materials mentioned herein are mentioned for the purpose of describing and disclosing the materials and/or methodologies which are reported in the publications which might be used in connection with the invention. Nothing herein is to be construed as an admission that the invention is not entitled to antedate such disclosure by virtue of prior invention.

Before the present compounds, compositions, articles, devices, and/or methods are disclosed and described, it is to be understood that this invention is not limited to specific synthetic methods, specific recombinant biotechnology methods unless otherwise specified, or to particular reagents unless otherwise specified, as such may, of course, vary. It is also to be understood that the terminology used herein is for the purpose of describing particular embodiments only and is not intended to be limiting.

Throughout this application, reference is made to various proteins and nucleic acids. It is understood that any names used for proteins or nucleic acids are art-recognized names, such that the reference to the name constitutes a disclosure of the molecule itself.

As used herein and in the appended claims, the singular forms "a," "and," and "the" include plural referents unless the context clearly dictates otherwise. Thus, for example, reference to "a method" includes a plurality of such methods and reference to "a dose" includes reference to one or more doses and equivalents thereof known to those skilled in the art, and so forth.

The term "about" or "approximately" means within an acceptable error range for the particular value as determined by one of ordinary skill in the art, which will depend in part on how the value is measured or determined, e.g., the limitations of the measurement system. For example, "about" can mean within 1 or more than 1 standard deviations per the practice in the art. Alternatively, "about" can mean a range of up to 20%, or up to 10%, or up to 5%, or up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

The term "antibody" is used in the broadest sense and specifically covers single monoclonal antibodies (including agonist and antagonist antibodies) and antibody compositions with polyepitopic specificity. It is also within the scope hereof to use natural or synthetic analogs, mutants, variants, alleles, homologs and orthologs (herein collectively referred to as "analogs") of the antibodies hereof as defined herein. Thus, according to one embodiment hereof, the term "antibody hereof' in its broadest sense also covers such analogs. Generally, in such analogs, one or more amino acid residues may have been replaced, deleted and/or added, compared to the antibodies hereof as defined herein.

"Antibody fragment", and all grammatical variants thereof, as used herein are defined as a portion of an intact antibody comprising the antigen binding site or variable region of the intact antibody, wherein the portion is free of the constant heavy chain domains (i.e. CH2, CH3, and CH4, depending on antibody isotype) of the Fc region of the intact antibody. Examples of antibody fragments include Fab, Fab', Fab'- SH, F(ab')₂, and Fv fragments; diabodies; any antibody fragment that is a polypeptide having a primary structure consisting of one uninterrupted sequence of contiguous amino acid residues (referred to herein as a "single-chain antibody fragment" or "single chain polypeptide"), including without limitation (1) single-chain Fv (scFv) molecules (2) single chain polypeptides containing only one light chain variable domain, or a fragment thereof that contains the three CDRs of the light chain variable domain, without an associated heavy chain moiety and (3) single chain polypeptides containing only one heavy chain variable region, or a fragment thereof containing the three CDRs of the heavy chain variable region, without an associated light chain moiety; and multispecific or multivalent structures formed from antibody fragments. In an antibody fragment comprising one or more heavy chains, the heavy chain(s) can contain any constant domain sequence (e.g. CHI in the IgG isotype) found in a non-Fc region of an intact antibody, and/or can contain any hinge region sequence found in an intact antibody, and/or can contain a leucine zipper sequence fused to or situated in the hinge region sequence or the constant domain sequence of the heavy chain(s). The term further includes single domain antibodies ("sdAB") which generally refers to an antibody fragment having a single monomeric variable antibody domain, (for example, from camelids). Such antibody fragment types will be readily understood by a person having ordinary skill in the art.

"Binding" refers to a sequence-specific, non-covalent interaction between macromolecules (e.g., between a protein and a nucleic acid). Not all components of a binding interaction need be sequence-specific (e.g., contacts with phosphate residues in a DNA backbone), as long as the interaction as a whole is sequence-specific.

A "binding protein" is a protein that is able to bind non-covalently to another molecule. A binding protein can bind to, for example, a DNA molecule (a DNA-binding protein), an RNA molecule (an RNA-binding protein) and/or a protein molecule (a protein-binding protein). In the case of a protein-binding protein, it can bind to itself (to form homodimers, homotrimers, etc.) and/or it can bind to one or more molecules of a different protein or proteins. A binding protein can have more than one type of binding activity. For example, zinc finger proteins have DNA-binding, RNA-binding and protein-binding activity.

As used herein, the term "comprising" is intended to mean that the compositions and methods include the recited elements, but do not exclude others. "Consisting essentially of' when used to define compositions and methods, shall mean excluding other elements of any essential significance to the combination when used for the intended purpose. Thus, a composition consisting essentially of the elements as defined herein would not exclude trace contaminants or inert carriers. "Consisting of shall mean excluding more than trace elements of other ingredients and substantial method steps. Embodiments defined by each of these transition terms are within the scope of this invention.

As used herein, the term "effector molecule" means a molecule, such as a protein or protein domain, oftentimes an enzymatic protein, capable of exerting a localized effect in a cell.. The effector molecule may take a variety of different forms, including selectively binding to a protein or to DNA, for example, to regulate a biological activity. Effector molecules may have a wide variety of different activities, including, but not limited to nuclease activity, increasing or decreasing enzyme activity, increasing or decreasing gene expression, or affecting cell signalling. Other examples of effector molecules will be readily appreciated by one having ordinary skill in the art.

As used herein, the term "epitope tag", or otherwise "affinity tag", refers to a short amino acid sequence or peptide enabling a specific interaction with a protein or a ligand.

As used herein, "epitope" refers to an antigenic determinant of a polypeptide. An epitope could comprise three amino acids in a spatial conformation, which is unique to the epitope. Generally, an epitope consists of at least 4, 5, 6, or 7 such amino acids, and more usually, consists of at least 8, 9, or 10 such amino acids. Methods of determining the spatial conformation of amino acids are known in the art, and include, for example, x-ray crystallography and two-dimensional nuclear magnetic resonance.

As used herein, "expression" refers to the process by which polynucleotides are transcribed into mRNA and/or the process by which the transcribed mRNA is subsequently being translated into peptides, polypeptides, or proteins. If the polynucleotide is derived from genomic DNA, expression may include splicing of the mRNA in an eukaryotic cell.

"Gene expression" refers to the conversion of the information, contained in a gene, into a gene product. A gene product can be the direct transcriptional product of a gene (e.g., mRNA, tRNA, rRNA, antisense RNA, ribozyme, shRNA, micro RNA, structural RNA or any other type of RNA) or a protein produced by translation of a mRNA. Gene products also include RNAs which are modified, by processes such as capping, polyadenylation, methylation, and editing, and proteins modified by, for example, methylation, acetylation, phosphorylation, ubiquitination, ADP-ribosylation, myristilation, and glycosylation.

"Modulation" or "regulation" of gene expression refers to a change in the activity of a gene. Modulation of expression can include, but is not limited to, gene activation and gene repression.

As used herein, the term "operatively linked" or its equivalents (e.g., "linked operatively") means two or more molecules are positioned with respect to each other such that they are capable of interacting to affect a function attributable to one or both molecules or a combination thereof.

The term "scFv" refers to a single-chain variable fragment. scFv is a fusion protein of the variable regions of the heavy (VH) and light chains (VL) of immunoglobulins, connected with a linker peptide. The linker peptide may be from about 5 to 40 amino acids or from about 10 to 30 amino acids or about 5, 10, 15, 20, 25, 30, 35, or 40 amino acids in length. Single-chain variable fragments lack the constant Fc region found in complete antibody molecules, and, thus, the common binding sites (e.g., Protein G) used to purify antibodies. The term further includes a scFv that is an intrabody, an antibody that is stable in the cytoplasm of the cell, and which may bind to an intracellular protein.

As used herein, the term "single domain antibody" means an antibody fragment having a single monomeric variable antibody domain which is able to bind selectively to a specific antigen. A single-domain antibody generally is a peptide chain of about 110 amino acids long, comprising one variable domain (VH) of a heavy-chain antibody, or of a common IgG, which generally have similar affinity to antigens as whole antibodies, but are more heat-resistant and stable towards detergents and high concentrations of urea. Examples are those derived from camelid or fish antibodies. Alternatively, single-domain antibodies can be made from common murine or human IgG with four chains.

The terms "specifically bind" and "specific binding" as used herein refer to the ability of an antibody, an antibody fragment or a nanobody to preferentially bind to a particular antigen that is present in a homogeneous mixture of different antigens. In certain embodiments, a specific binding interaction will discriminate between desirable and undesirable antigens in a sample, in some embodiments more than about ten- to 100-fold or more (e.g., more than about 1000- or 10,000-fold). "Specificity" refers to the ability of an immunoglobulin or an immunoglobulin fragment, such as a nanobody, to bind preferentially to one antigenic target versus a different antigenic target and does not necessarily imply high affinity.

A "target site" or "target sequence" is a nucleic acid sequence that defines a portion of a nucleic acid to which a binding molecule will bind, provided sufficient conditions for binding exist.

Additional advantages of the invention will be set forth in part in the description which follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

Disclosed herein are compositions and methods for addressing one or more of the aforementioned problems in the art. In one aspect, non-covalently linked components and methods of making and using non-covalently linked components, are disclosed. The various components may take a variety of different forms as described herein. For example, non-covalently linked (i.e., operatively linked) proteins may be used to allow temporary interactions that avoid one or more problems in the art. The ability of non-covalently linked components, such as proteins, to associate and dissociate enables a functional association only or primarily under circumstances where such association is needed for the desired activity. The linkage may be of duration sufficient to allow the desired effect.

In one aspect, a method for directing proteins to a specific locus in a genome of an organism is disclosed. The method may comprise the steps of providing a DNA localization component and providing an effector molecule, wherein the DNA localization component and the effector molecule are capable of operatively linking via a non-covalent linkage.

### DNA Localization Component

In one aspect, the DNA localization component may be capable of binding a specific DNA sequence. The DNA localization component may be selected from, for example, a DNA-binding oligonucleotide, a DNA-binding protein, a DNA binding protein complex, and combinations thereof. Other suitable DNA binding components will be recognized by one of ordinary skill in the art.

In one aspect, the DNA localization component may comprise an oligonucleotide directed to a specific locus or loci in the genome. The oligonucleotide may be selected from DNA, RNA, DNA/RNA hybrids, and combinations thereof.

In one aspect, the DNA localization component may comprise a nucleotide binding protein or protein complex that binds an oligonucleotide when bound to a target DNA. The protein or protein complex may be capable of recognizing a feature selected from RNA-DNA heteroduplexes, R-loops, or combinations thereof. In one aspect, the DNA localization component may comprise a protein or protein complex capable of recognizing an R-loop selected from Cas9, Cascade complex, RecA, RNase H, RNA polymerase, DNA polymerase, or a combination thereof.

In one aspect, the DNA localization component may comprise an engineered protein capable of binding to target DNA. In this aspect, the DNA localization component may comprise a protein capable of binding a DNA sequence selected from meganuclease, zinc finger array, transcription activator-like (TAL) array, and combinations thereof.

In other aspects, the DNA localization component may comprise a protein that contains a naturally occurring DNA binding domain. The DNA localization component may comprise, for example, a protein comprising a naturally occurring DNA binding domain is selected from a bZIP domain, a Helix-loop-helix, a Helix-turn-helix, a HMG-box, a Leucine zipper, a Zinc finger, or a combination thereof.

### Effector Molecule

In one aspect, the method comprises providing an effector molecule.

In one aspect, the effector molecule may be selected from a transcription factor (activator or repressor), chromatin remodeling factor, exonuclease, endonuclease, transposase, methytransferase, demethylase, acetyltransferase, deacetylase, kinase, phosphatase, integrase, recombinase, ligase, topoisomerase, gyrase, helicase, fluorophore, and combinations thereof.

In one aspect, the effector molecule may comprise a nuclease. The nuclease may be any nuclease readily appreciated by one of skill in the art. Suitable nucleases include, for example, a restriction endonuclease, homing endonuclease, S1 Nuclease, mung bean nuclease, pancreatic DNase I, micrococcal nuclease, yeast HO endonuclease, or a combination thereof. In one aspect, the effector molecule may comprise a Type IIS restriction endonuclease. For example, in some aspects, the effector molecule may comprise an endonuclease selected from AciI, Mn1I, AlwI, BbvI, BccI, BceAI, BsmAI, BsmFI, BspCNI, BsrI, BtsCI, HgaI, HphI, HpyAV, MbolI, MylI, PleI, SfaNI, AcuI, BciVI, BfuAI, BmgBI, Bmrl, BpmI, BpuEI, BsaI, BseRI, BsgI, BsmI, BspMI, BsrBI, BsrBI, BsrDI, BtgZI, BtsI, Earl, EciI, MmeI, NmeAIII, BbvCI, Bpu10I, BspQI, SapI, BaeI, BsaXI, CspCI, Fokl, BfiI, MboII, Acc36I and Clo051. In other aspects, the effector molecule may comprise a PB transposase (PBase).

In one aspect, the effector molecule may be an endonuclease. In certain embodiments, the effector molecule may be Fokl. In certain embodiments, the effector molecule may be BfiI. In certain embodiments, the effector molecule may be BmrI. In certain embodiments, the effector molecule may be Clo051.

### Linkage

In one aspect, the method may comprise a non-covalent linkage between the DNA localization component and the effector molecule. The non-covalent linkage may comprise an antibody, an antibody fragment, an antibody mimetic, or a scaffold protein.

Antibodies and fragments thereof, include, but are not limited to, single-chain variable fragment (scFv), single domain antibodies (sdAB), monobodies, and nanobodies. For example, the non-covalent linkage may comprise, a single-chain variable fragment (scFv) or a single domain antibody (sdAB) covalently attached to one or more effector molecules, and which is capable of a non-covalent association to the DNA localization component. In a further aspect, the non-covalent linkage may comprise a single-chain variable fragment (scFv) covalently attached to the DNA localization component and which non-covalently binds directly to the effector component. In a further aspect, the non-covalent linkage may comprise a single-chain variable fragment (scFv) covalently attached to either the effector molecule or the DNA localization component. The scFV may then non-covalently bind to an epitope tag covalently attached to the opposite component (i.e., to the DNA localization component or the effector molecule).

The non-covalent linkage may comprise, for example, an antibody mimetic. As used herein, the term "antibody mimetic" is intended to describe an organic compound that specifically binds a target sequence and has a structure distinct from a naturally-occurring antibody. Antibody mimetics may comprise a protein, a nucleic acid, or a small molecule. The target sequence to which an antibody mimetic of the disclosure specifically binds may be an antigen. Antibody mimetics may provide superior properties over antibodies including, but not limited to, superior solubility, tissue penetration, stability towards heat and enzymes (e.g. resistance to enzymatic degradation), and lower production costs. Exemplary antibody mimetics include, but are not limited to, an affibody, an afflilin, an affimer, an affitin, an alphabody, an anticalin, and avimer (also known as avidity multimer), a DARPin (Designed Ankyrin Repeat Protein), a Fynomer, a Kunitz domain peptide, and a monobody.

Affibody molecules of the disclosure comprise a protein scaffold comprising or consisting of one or more alpha helix without any disulfide bridges. Preferably, affibody molecules of the disclosure comprise or consist of three alpha helices. For example, an affibody molecule of the disclosure may comprise an immunoglobulin binding domain. An affibody molecule of the disclosure may comprise the Z domain of protein A.

Affilin molecules of the disclosure comprise a protein scaffold produced by modification of exposed amino acids of, for example, either gamma-B crystallin or ubiquitin. Affilin molecules functionally mimic an antibody's affinity to antigen, but do not structurally mimic an antibody. In any protein scaffold used to make an affilin, those amino acids that are accessible to solvent or possible binding partners in a properly-folded protein molecule are considered exposed amino acids. Any one or more of these exposed amino acids may be modified to specifically bind to a target sequence or antigen.

Affimer molecules of the disclosure comprise a protein scaffold comprising a highly stable protein engineered to display peptide loops that provide a high affinity binding site for a specific target sequence. Exemplary affimer molecules of the disclosure comprise a protein scaffold based upon a cystatin protein or tertiary structure thereof. Exemplary affimer molecules of the disclosure may share a common tertiary structure of comprising an alpha-helix lying on top of an anti-parallel beta-sheet.

Affitin molecules of the disclosure comprise an artificial protein scaffold, the structure of which may be derived, for example, from a DNA binding protein (e.g. the DNA binding protein Sac7d). Affitins of the disclosure selectively bind a target sequence, which may be the entirety or part of an antigen. Exemplary affitins of the disclosure are manufactured by randomizing one or more amino acid sequences on the binding surface of a DNA binding protein and subjecting the resultant protein to ribosome display and selection. Target sequences of affitins of the disclosure may be found, for example, in the genome or on the surface of a peptide, protein, virus, or bacteria. In certain embodiments of the disclosure, an affitin molecule may be used as a specific inhibitor of an enzyme. Affitin molecules of the disclosure may include heat-resistant proteins or derivatives thereof.

Alphabody molecules of the disclosure may also be referred to as Cell-Penetrating Alphabodies (CPAB). Alphabody molecules of the disclosure comprise small proteins (typically of less than 10 kDa) that bind to a variety of target sequences (including antigens). Alphabody molecules are capable of reaching and binding to intracellular target sequences. Structurally, alphabody molecules of the disclosure comprise an artificial sequence forming single chain alpha helix (similar to naturally occurring coiled-coil structures). Alphabody molecules of the disclosure may comprise a protein scaffold comprising one or more amino acids that are modified to specifically bind target proteins. Regardless of the binding specificity of the molecule, alphabody molecules of the disclosure maintain correct folding and thermostability.

Anticalin molecules of the disclosure comprise artificial proteins that bind to target sequences or sites in either proteins or small molecules. Anticalin molecules of the disclosure may comprise an artificial protein derived from a human lipocalin. Anticalin molecules of the disclosure may be used in place of, for example, monoclonal antibodies or fragments thereof. Anticalin molecules may demonstrate superior tissue penetration and thermostability than monoclonal antibodies or fragments thereof. Exemplary anticalin molecules of the disclosure may comprise about 180 amino acids, having a mass of approximately 20 kDa. Structurally, anticalin molecules of the disclosure comprise a barrel structure comprising antiparallel beta-strands pairwise connected by loops and an attached alpha helix. In preferred embodiments, anticalin molecules of the disclosure comprise a barrel structure comprising eight antiparallel beta-strands pairwise connected by loops and an attached alpha helix.

Avimer molecules of the disclosure comprise an artificial protein that specifically binds to a target sequence (which may also be an antigen). Avimers of the disclosure may recognize multiple binding sites within the same target or within distinct targets. When an avimer of the disclosure recognize more than one target, the avimer mimics function of a bi-specific antibody. The artificial protein avimer may comprise two or more peptide sequences of approximately 30-35 amino acids each. These peptides may be connected via one or more linker peptides. Amino acid sequences of one or more of the peptides of the avimer may be derived from an A domain of a membrane receptor. Avimers have a rigid structure that may optionally comprise disulfide bonds and/or calcium. Avimers of the disclosure may demonstrate greater heat stability compared to an antibody.

DARPins (Designed Ankyrin Repeat Proteins) of the disclosure comprise genetically-engineered, recombinant, or chimeric proteins having high specificity and high affinity for a target sequence. In certain embodiments, DARPins of the disclosure are derived from ankyrin proteins and, optionally, comprise at least three repeat motifs (also referred to as repetitive structural units) of the ankyrin protein. Ankyrin proteins mediate high-affinity protein-protein interactions. DARPins of the disclosure comprise a large target interaction surface.

Fynomers of the disclosure comprise small binding proteins (about 7 kDa) derived from the human Fyn SH3 domain and engineered to bind to target sequences and molecules with equal affinity and equal specificity as an antibody.

Kunitz domain peptides of the disclosure comprise a protein scaffold comprising a Kunitz domain. Kunitz domains comprise an active site for inhibiting protease activity. Structurally, Kunitz domains of the disclosure comprise a disulfide-rich alpha+beta fold. This structure is exemplified by the bovine pancreatic trypsin inhibitor. Kunitz domain peptides recognize specific protein structures and serve as competitive protease inhibitors. Kunitz domains of the disclosure may comprise Ecallantide (derived from a human lipoprotein-associated coagulation inhibitor (LACI)).

Monobodies of the disclosure are small proteins (comprising about 94 amino acids and having a mass of about 10 kDa) comparable in size to a single chain antibody. These genetically engineered proteins specifically bind target sequences including antigens. Monobodies of the disclosure may specifically target one or more distinct proteins or target sequences. In preferred embodiments, monobodies of the disclosure comprise a protein scaffold mimicking the structure of human fibronectin, and more preferably, mimicking the structure of the tenth extracellular type III domain of fibronectin. The tenth extracellular type III domain of fibronectin, as well as a monobody mimetic thereof, contains seven beta sheets forming a barrel and three exposed loops on each side corresponding to the three complementarity determining regions (CDRs) of an antibody. In contrast to the structure of the variable domain of an antibody, a monobody lacks any binding site for metal ions as well as a central disulfide bond. Multispecific monobodies may be optimized by modifying the loops BC and FG. Monobodies of the disclosure may comprise an adnectin.

The non-covalent linkage may comprise, for example, a scaffold protein. Scaffold proteins of the disclosure include, for example, antibody mimetics of the disclosure. Scaffold proteins of the disclosure further include, for example, small modular immunopharmaceutical (SMIP) molecules, a domain antibody, and a nanobody.

SMIP molecules of the disclosure are artificial proteins comprising one or more sequences or portions of an immunoglobulin (antibody) that are monospecific for a target sequence or antigen. SMIPs of the disclosure may substitute for the use of a monoclonal antibody. Structurally, SMIPs are single chain proteins comprising a binding region, a hinge region (i.e. a connector), and an effector domain. The binding region of a SMIP may comprise a modified single-chain variable fragment (scFv). SMIPs may be produced from genetically-modified cells as dimers.

Domain antibodies of the disclosure comprise a single monomeric variable antibody domain (i.e. either heavy or light variable domain). Domain antibodies of the disclosure demonstrate the same antigen specificity as a whole and intact antibody. Domain antibodies of the disclosure may be manufactured, at least in part, by immunization of dromedaries, camels, llamas, alpacas or sharks with the desired antigen and subsequent isolation of the mRNA coding for heavy-chain antibodies.

Nanobodies of the disclosure comprise a VHH single domain antibody. Nanobodies of the disclosure may comprise single domain antibodies of the disclosure.

In one aspect, the non-covalent linkage may comprise a protein binding domain covalently attached to either the effector molecule or the DNA localization component and which is capable of a non-covalent interaction with the opposite component. Non-limiting examples of protein binding domains include, for example, SH2, SH3, PTB, LIM, SAM, PDZ, FERM, CH, Pleckstin, WW, WSxWS, and the E3 ligase domain.

In one aspect, the non-covalent linkage may comprise a protein covalently attached to either the effector molecule or the DNA localization component that is capable of binding to a protein covalently attached to the opposite component. Non-limiting examples include any two proteins that interact non-covalently. Such proteins are readily identified via the Database of Interacting Proteins (DIP), STRING, BioGRID, MIPS, or the like.

In one aspect, the non-covalent linkage may comprise a small molecule covalently attached either to an effector molecule or a DNA localization component, and is capable of forming a non-covalent bond to a protein or other small molecule covalently attached to the opposite component. One such example would include biotin attached to an oligonucleotide and avidin covalently linked to an effector molecule.

The above described methods and compositions may be used, for example, in situations in which a particular protein may have several functions. Transposase proteins, for example, must perform several steps to achieve the desired function, including transposon recognition, cleavage of DNA to excise a transposon, movement of a transposon sequence to a new genomic location, recognition of a new target site, and cleavage of DNA to integrate the transposon at a new locus. In certain aspects, it may be desirable to direct a transposase to integrate a transposon at a particular site in the genome. In these aspects, this could be carried out by, for example, adding a heterologous protein with site-specific DNA binding activity. However, the heterologous protein with site-specific DNA binding activity would only be required during the target site recognition step, and the presence of this protein at earlier stages in the process described above may be detrimental to the other steps. As such, in this aspect, a temporary association of the heterologous protein with site-specific DNA binding activity with the transposase would allow the transposase to be directed to the genomic site of interest while allowing for the other steps of the process to be carried out with limited interference of the protein due to the non-covalent binding.

As another example, it may be desirable to have an enzymatic protein, such as a nuclease, methylase, deacetylase, etc. to temporarily interact with a specific DNA binding domain so that its activity occurs at a specific location in the genome. For example, it may be desired to cause a Fokl restriction nuclease to temporarily interact with a Cas9 protein that is catalytically inactive for DNA cleavage.

In one aspect, the linker comprises a non-covalent linkage between the DNA binding element and the effector. For example, in one aspect, phage display (PhD) may be used to produce single-chain variable fragment (scFv) antibodies or single domain antibodies (sdAbs) against a particular target. PhD may be used to identify a scFv antibody against an effector, for example piggyBBac (PB) transposase that provides a linkage. A large diversity in scFv affinity may be obtained by limiting the stringency of the affinity selection process. In one aspect, the linkage may be between PB transposase (PBase) and a modular DNA binding domain such as a polydactyl zinc finger, a TAL array, or a dCas9 protein (with associated guide RNA). In some aspects, a scFv antibody with a faster off-rate may provide permissive "breathing" of the complex. In other aspects, conformation and/or flexibility of an effector and DNA binding element may be critical. Non-covalent linkages may provide conformational pliability to the disclosed gene editing compositions. Alternatively, slower off-rates (and a higher Kd) of an scFv that binds particular epitopes of an effector may provide an optimal stability and conformation of the gene editing complex that would not otherwise be obtainable through traditional peptide linkage. A near-exhaustive search among scFv antibodies allows one to select from among a large diversity of possible conformations of a gene editing complex. A PhD strategy creates such diversity through the generation of unique monovalent scFvs against multiple unique epitopes.

Furthermore, a non-covalent linkage method, such as that achieved through the use of a scFv antibody, may employ an unmodified and native effector (e.g., PB). This provides a reversible associate between the effector and the DNA binding element, which may circumvent any permanent interference with the activity of an effector that may occur when it is subjected to covalent linkage. Certain non-covalent associations could introduce steric hindrances that compromise the effector reaction. As several activities may be involved (site recognition, strand cleavage, transposon binding and integration) it is likely that each separate step may be differentially affected by a particular steric hindrance. For example, if transposase association with the DNA transposon (during transposon mobilization from one genomic site to another) has a very slow off-rate, then it would be detrimental to have a very high affinity association between a DNA binding element-scFv and the PBase that disrupts this association. However, if the DNA binding element-scFv protein binds with a lower, but significant affinity, it could be temporarily displaced during transposon mobilization. It is possible that such an early step could involve temporary dissociation of DNA binding factor-scFv with the PBase, with subsequent reassembly of the complex at later steps to create a fully functional and DNA binding factor-enabled site-specific transposase.

### Examples

Phage display is used to identify an scFv antibody against PBase that provides an optimal linkage. A large diversity in scFv affinity can be obtained by limiting the stringency of the affinity selection process. This diversity may represent a key advantage of a PhD approach for identifying a successful linkage between PBase and a modular DNA binding protein (DBP). In some instances, an scFv antibody with a faster off-rate may provide permissive "breathing" of the DBP-PBase complex. Previous studies show that, when E2c is fused to the SB transposase, the efficiency is almost doubled if there is complete mismatch in one half-site of the 18 bp recognition sequence [36]. Even though a "flexible" 15-residue linker is used (-GGS5-) for SB-E2c fusion, it has been hypothesized that the flexibility provided by E2c half-site recognition enables efficient site-specific transposition. This may also be true for fusions with PBase. Regardless, the conformation and/or flexibility of the DBP and fused transposase appear critical, and a non-covalent linkage may provide this conformational pliability. Alternatively, slower off-rates (and a higher Kd) of an scFv that binds particular epitopes of PBase may provide an optimal stability and conformation of the DBP-PBase complex-a conformation otherwise not attainable through simple peptide linkage. A near-exhaustive search among scFv antibodies allows one to select from among a large diversity of possible conformations of DBP-PBase complexes. A PhD strategy may create such diversity through the generation of unique monovalent scFvs against multiple unique epitopes.

A non-covalent linkage method, such as that achieved through the use of an scFv antibody employs an unmodified and native PBase protein. This is believed to provide a reversible association between PBase and the DBP, which may circumvent any permanent interference with PBase catalytic activity that may occur when it is subjected to covalent linkage. Certain non-covalent associations could introduce steric hindrances that compromise the transposase reaction, but since the transposition reaction involves separate catalytic steps (site recognition, strand cleavage, transposon binding, and integration), it is likely that each separate step would be differentially affected by a particular steric hindrance. For example, if transposase association with the DNA transposon (during transposon mobilization from one genomic site to another) has a very slow off-rate, then it would be clearly detrimental to have a very high affinity association between E2c-scFv and the PBase that disrupts this association. However, if the E2c-scFv protein binds with a lower, but significant affinity, it could be temporarily displaced during transposon mobilization. It is possible that such an early step could involve temporary dissociation of E2c-scFv with the PBase, with subsequent reassembly of the complex at later steps to create a fully functional and E2c-enabled site-specific transposase.

### Immunization for producing anti-PB antibodies.

An antibody library is produced from immunized rabbits using methods well known in the art. Rabbits provide two key advantages: 1) their size provides large amounts of tissue (spleen and bone marrow) and ample serum for titering and 2) fewer PCR primers are needed for antibody gene amplification since fewer gene segments are rearranged during B-cell development in rabbits. Six New Zealand White rabbits are immunized each with 200 pg of recombinant PBase protein plus adjuvant, and serum is collected six weeks after immunization for determining antibody titers. Titers are determined by ELISAs on immobilized recombinant PBase protein and the animals with the highest titers (at least 1:1000) are sacrificed for isolating the spleen and bone marrow. If rabbits do not produce sufficient titers, a naive library from embryonic rabbit tissue is used. This provides an unbiased collection of un-rearranged heavy and light chain genes. Total RNA will be extracted from tissues using Trizol (Invitrogen), and cDNA synthesis is performed with the iScript cDNA synthesis kit (BioRad).

### Generating scFv gene fusions.

To isolate expressed variable regions of heavy and light chain genes from rabbit, several primers are used. Eight primers are used for kappa and lambda light chain amplification and five primers are used for heavy chain gene amplification. Primers also contain the coding sequence for an 18 amino acid linker sequence (SSGGGGSGGGGGGSSRSS) (SEQ ID NO: 2), which links the variable regions of the heavy and light chains (VH and VL). This longer linker sequence provides better stability of monomeric forms of scFv fragments. The PCR products of the VH and VL genes overlap in this linker region and can then be assembled by overlap-extension (OLE) PCR (FIG 1). PCR products are then digested with Sfil, ligated with Sfil-digested pComb3H, and DNA will then be size-selected by gel electrophoresis. This plasmid enables phagemid display of an scFv fused to the pill coat protein. About 5 molecules of pill phage coat protein is present on each phage particle. The pComb3H plasmid expresses the scFv-plll fusion at a level such that about one or two molecules are integrated with wild-type pill (which is provided by helper phage). Since up to 1012 phage particles can be generated in a single preparation, a very large number of scFvs can thus be screened. In PhD the scFv coding sequence is always linked to the phage particle displaying the protein, so subsequent DNA sub-cloning is conveniently achieved.

### Producing and screening the phage library.

Ligated plasmid DNA (50 to 100 ng) is electroporated into ER2538 E. coli (New England Biolabs). E. coli will then be recovered by shaking for 1 hour at 37°C in 5 mLs of SOC. Phage is produced with the VCSM13 helper phage, which has a defective origin of replication. Phage particles will be precipitated with PEG-8000 and then isolated by further centrifugation. This phage prep is the primary library, and will be affinity selected by "panning." Double recognition panning is performed in which the phage elution is re-incubated with the immobilized antigen, washed, and eluted again. This helps eliminate non-specific phage. To test each round of selection, phage pools are assayed by ELISAs for affinity to the PBase antigen. PBase or BSA are coated to 96-well plates, incubated with phage, and then incubated with a horseradish peroxidase (HRP) conjugated anti-M13 antibody, which recognizes the M13 phage coat protein. An increasing ELISA titer indicates successful affinity selection of each phage pool.

### Transferring the scFv library into a lentiviral vector, and expansion in E. coli.

Phagemid DNA is isolated from bacteria after the 2nd (R2) and 3rd (R3) rounds of panning by infecting E. coli with each phage pool, selecting with carbenicillin, followed by standard plasmid preparation. Plasmid DNA is digested with Sfi1 to liberate the scFv coding sequence, and ligated upstream of the E2c coding sequence within the pLVX-IRES-ZsGreen1 (Clontech) vector. The E2c coding sequence also has a short linker sequence (GGSSRSS) (SEQ ID NO: 3) and creates a fusion of the scFv library to the N-terminal portion of E2c. The two ensuing plasmid libraries (R2 and R3) will then be prepared as in Aim 2, for production of two lentivirus libraries.

### Lentivirus library production.

For production of lentivirus particles, the Lenti-X HT Packaging System (Clontech) is used, which produces viral titers as high as 5x10⁸ infectious units per mL. Virus is produced according to the manufacturer's specifications. Viral supernatants are titered on HepG2 and Huh7 cells, followed by FACS fluorescence produced by the ZsGreenl reporter to count transduced cells.

HEK293 cells are also be infected with viral supernatants to determine the ability of scFv-E2c fusion proteins to bind the E2c target sequence, to ensure there is no loss of binding affinity of the E2c domain. Nuclear lysates will be prepared from transduced cells and used for electrophoretic mobility shift assays (EMSAs) with labeled DNA containing the E2c target sequence. The affinity is compared to nuclear lysates from cells transduced for expression of an unmodified E2c. Since this procedure will screen a mixture of E2c fusion proteins (from the library), the affinities will represent an average for the library-some fusions may have compromised affinities, while others may not. The objective is to ensure that the overall average affinity is not dramatically reduced (by 50%), which would otherwise indicate that the fusion process itself has adversely affected E2c affinity. Affinities will be calculated as understood in the art.

### Screening strategy.

To screen for effective site-specific integration, a puromycin acetyltransferase (PAC) complementation strategy in which site-specific integration yields a functional PAC transgene is used. Similar strategies have been used to detect chromosomal translocations. This selection is achieved by separating the PAC coding region into two separate sequences that can be linked through splicing. The first component (E2c-SA-PAC141) consists of a 3' fragment of the PAC open reading frame (ORF) that encodes the C-terminal 141 amino acids immediately downstream of the splice acceptor (SA) from the intronl/exon2 boundary of the Adenovirus II (Ad2) late major transcript (FIG 2). The SV40 late polyadenylation signal, providing a transcript termination signal, is located just 3' of the PAC ORF fragment (PAC141). An E2c recognition sequence is inserted within a 10.47 Kb fragment of the p53 intron, which lacks splice donor and acceptor sequences, which is flanked by two identical but inverted copies of the SA-PC141 fragment (FIG 2). This arrangement enables splicing, complementation, and PAC expression following a site-specific insertion in either orientation. A large intron fragment is used because it is likely deficient for cryptic splice sites. Stable HepG2 and Huh7 cell lines containing the E2c-SA-PAC141 cassette are generated by co-transfecting cells with a hygromycin resistance cassette driven by the thymidine kinase promoter (TK-Hygro), followed by selection with hygromycin. Stable lines are treated with puro to ensure sensitivity to this antibiotic.

The 5' portion of the PAC gene (PAC58), containing the remaining coding sequence of the PAC ORF, is mobilized via PB into a region between the PAC141 sequences by site-specific integration near the E2c sequence (FIG 2). This PB transposon (B11-iPAC58-SD) has a cDNA containing the 5' coding region of the PAC gene instead of the EGFP-IRES sequence. The expression of a functional PAC transcript is facilitated by splicing between the Ad2 acceptor and donor sites. These potent splice sites do not undergo alternative splicing.

### Selection for site-specific integration.

Sixty million cells from HepG2 or Huh7 stable cell lines are transduced in 10 x 10 cm dishes at a multiplicity of infection (M01) of one with each of the ten retroviral libraries (eight linker libraries and two scFv libraries). HepG2 and Huh7 can be transduced with lentivirus (LV) vectors at an efficiency of about 30% to 70% and 70% to 95%, respectively. LV infection of these hepatocyte cell lines does not appear to compromise the hepatocyte phenotype. Cells receiving the scFv lentiviral library are co-transduced with lentivirus generated with the PBase coding sequences. Twenty-four hours later, the medium is changed and cells incubated for an additional 24 hours, then transfected with a plasmid containing the BII-iPAC58-SD transposon. The transposon is supplied as transfected DNA because in all likelihood, for actual gene therapy, DNA will be delivered either by liposomes, nanoparticles, or adenovirus in the form of a DNA episome. Cells are then incubated for an additional 72 hours followed by selection with puro for 48 hours. This will select for site-specific integration of the BII-iPAC58-SD transposon upstream of the E2c-SA-PAC141 cassette. Multiple cell lines (2 to 3) for HepG2 and Huh7 will be screened to account for different genomic contexts of the E2c-SA-PAC141 transgene. Transduction with PBase alone, via lentivirus, (without E2c or E2c-scFv) will represent the negative control, and will likely yield few puro-resistant cells, if any.

### Identifying and testing the effective linkage(s).

Genomic DNA from puro-resistant cells is isolated for PCR using primers that flank the library cloning sites. These cells will generally contain a site-specific BIIiPAC58-SD transposon integration upstream of the E2c-SA-PAC141 cassette. Among these cells, many will contain proviral DNA with specific linker or scFv antibody sequences that will have facilitated PB-mediated site-specific integration. To further enrich for the most efficient linkage strategy (whether covalent or non-covalent), a secondary library is generated from PCR-amplified linker or scFv sequences by digesting PCR products with Sfil and repeating the library production and selection, as above. After screening three library generations (GO, G1, G2) for each of ten (8 peptide linker libraries, and 2 scFv libraries) retroviral preps, the final PCR-amplified proviral insertions are cloned and sequenced to identify the linkers and/or scFv antibodies that yield efficient site-specific targeting. Testing is performed by assessing the efficiency of integration, as measured by the number of puroresistant cells obtained through transient transfection and PAC complementation. Linkage strategies identified are cloned and tested individually in the PAC complementation assay in HepG2 and Huh7 stable cell lines containing the E2c-SA-PAC141 cassette. PB-linker-E2c and E2c-scFv clones are inserted into pcDNA3.1 (Invitrogen) for transient transfection and expression. For E2c-scFv clones, the PBase in pcDNA3.1 is co-transfected with the pcDNA3.1-scFv plasmid to provide the PBase protein target. The BII-iPAC58-SD transposon is also supplied as plasmid DNA via co-transfection. Approximately 0.5-1 x 10⁶ cells are transfected in 6-well plates along with an equal amount of a CMV-GFP plasmid to determine transfection efficiency, as assessed at 48 hours by fluorescent microscopy. After 72 hours cells will be split into 10 cm dishes and puro added, and resistant colonies will be counted after one week of selection.

### Determining Off-Target Frequency.

To determine off-target frequency, non-specific insertions are quantified by Southern blot and QPCR. Ten puro-resistant colonies generated by the best variants (PB-linker-E2c or E2c-scFv clone, as identified above) are expanded and gDNA extracted. Southern blots are performed by digesting gDNA with BsrGI (for the ERBB2 locus) or PacI + SacI (for the E2c-SA-PAC141 cassette). DNA is probed with unique sequences within the p53 locus or ERBB2 gene. Fragments lacking an insertion are approximately 5 Kb for either p53 or ERBB2, while BII-iPAC58-SD integrations will add 2.4 Kb for each transposon insertion. Bands on the Southern blot, up to about 15 Kb, may be discernible, representing four transposon insertions for either the p53 intron or ERBB2 5'UTR target sites. This method cannot distinguish between the endogenous p53 genomic fragment and the p53 fragment in the E2c-SA-PAC141 cassette. However, the endogenous p53 intron region represents 11625000 of the haploid genome; the remaining 99.99984% of the genome is still measurable in the assay. Total copy number is determined by QPCR of gDNA with primers specific to the BII-iPAC58-SD transposon, along with copy number standards. This allows one to calculate the ratio of site-specific insertions to total insertions. A variant that yields 10-25% site-specific insertions is identified. Successful enrichment for efficient site-specific integration will be evident by an increasing number of resistant cells in each round of puro selection. It is possible that some puro-resistant cells could result from non-specific integration followed by chromosomal translocations. This would be rare and site-specific.

The practice of the present invention will employ, unless otherwise indicated, conventional techniques of molecular biology (including recombinant techniques), microbiology, cell biology and biochemistry, which are within the skill of the art.

All percentages and ratios are calculated by weight unless otherwise indicated.

All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "20 mm" is intended to mean "about 20 mm."

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a referenced document, the meaning or definition assigned to that term in this document shall govern.

## Claims

1. A method of modifying a genome of an organism *in vitro* or *ex vivo* comprising the steps of:
a. providing to a cell a DNA localization component that is capable of binding a specific DNA sequence; and
b. providing to the cell an endonuclease;
wherein said DNA localization component and said endonuclease are capable of operatively linking via a non-covalent linkage,
wherein the non-covalent linkage comprises a single domain antibody covalently attached to the DNA localization component and wherein the single domain antibody non-covalently binds to an epitope tag covalently bound to the endonuclease in the interior of the cell; or
wherein the non-covalent linkage comprises a single domain antibody covalently attached to the endonuclease and wherein the single domain antibody non-covalently binds directly to an epitope tag covalently bound to the DNA localization component in the interior of the cell;
and wherein the genome is not the germ line genome of a human.

2. The method of claim 1 wherein said DNA localization component is selected from a DNA-binding oligonucleotide, a DNA-binding protein, a DNA binding protein complex, and combinations thereof.

3. The method of claim 1 wherein said DNA localization component comprises an oligonucleotide directed to said specific loci in the genome; or an oligonucleotide, wherein said oligonucleotide is selected from DNA, RNA, DNA/RNA hybrids, and combinations thereof.

4. The method of claim 1 wherein said DNA localization component comprises a protein or a protein complex capable of recognizing a feature selected from RNA-DNA heteroduplexes, R-loops, and combinations thereof; or a protein or protein complex, wherein said protein or protein complex is capable of recognizing an R-loop selected from Cas9, Cascade complex, RecA, RNase H, RNA polymerase, DNA polymerase, and combinations thereof; or a protein capable of binding a DNA sequence selected from meganuclease, Zinc Finger array, TAL array, and combinations thereof.

5. The method of claim 1 wherein said DNA localization component comprises a protein comprising a naturally occurring DNA binding domain; or a protein comprising a naturally occurring DNA binding domain wherein said naturally occurring DNA binding domain is selected from a bZIP domain, a Helix-loop-helix, a Helix-turn-helix, a HMG-box, a Leucine zipper, a Zinc finger, and combinations thereof.

6. The method of claim 1 wherein said DNA localization component comprises an oligonucleotide directed to a target location in a genome; and a protein capable of binding to a target DNA sequence.

7. The method of claim 1 wherein said endonuclease is a restriction endonuclease, homing endonuclease, SI Nuclease, mung bean nuclease, pancreatic DNase I, micrococcal nuclease, yeast HO endonuclease, or a combination thereof.

8. The method of claim 1 wherein said endonuclease comprises a Type IIS restriction endonuclease.

9. The method of claim 1 wherein said endonuclease comprises an endonuclease selected from the group consisting of AciI, Mn1I, AlwI, BbvI, BccI, BceAI, BsmAI, BsmFI, BspCNI, BsrI, BtsCI, HgaI, HphI, HpyAV, MbolI, Myll, PleI, SfaNI, AcuI, BciVI, BfuAI, BmgBI, BmrI, BpmI, BpuEI, BsaI, BseRI, BsgI, BsmI, BspMI, BsrBI, BsrBI, BsrDI, BtgZI, BtsI, Earl, EciI, MmeI, NmeAIII, BbvCI, Bpu10I, BspQI, SapI, BaeI, BsaXI, CspCI, FokI, BfiI, MboII, Acc36I and Clo051.

10. The method of claim 1 wherein said endonuclease comprises BmrI, BfiI, Clo051, FokI or a transposase.

## Patentansprüche

1. Verfahren zum Modifizieren eines Genoms eines Organismus *in vitro* oder *ex vivo,* umfassend die Schritte:
a. Bereitstellen einer DNA-Lokalisierungskomponente, die dazu fähig ist, eine spezifische DNA-Sequenz zu binden, an eine Zelle und
b. Bereitstellen einer Endonuklease an die Zelle;
wobei die DNA-Lokalisierungskomponente und die Endonuklease dazu fähig sind, sich mittels einer nichtkovalenten Verknüpfung operativ zu verknüpfen,
wobei die nichtkovalente Verknüpfung einen Einzeldomänenantikörper umfasst, der kovalent an die DNA-Lokalisierungskomponente angelagert ist, und wobei der Einzeldomänenantikörper sich nichtkovalent an ein Epitop-Tag bindet, das kovalent an die Endonuklease im Inneren der Zelle gebunden ist; oder
wobei die nichtkovalente Verknüpfung einen Einzeldomänenantikörper umfasst, der kovalent an die Endonuklease angelagert ist, und wobei der Einzeldomänenantikörper sich nichtkovalent direkt an ein Epitop-Tag bindet, das kovalent an die DNA-Lokalisierungskomponente im Inneren der Zelle gebunden ist;
und wobei das Genom nicht das Keimbahngenom eines Menschen ist.

2. Verfahren nach Anspruch 1, wobei die DNA-Lokalisierungskomponente aus einem DNA-bindenden Oligonukleotid, einem DNA-bindenden Protein, einem DNA-bindenden Proteinkomplex und Kombinationen davon ausgewählt ist.

3. Verfahren nach Anspruch 1, wobei die DNA-Lokalisierungskomponente ein Oligonukleotid, das auf die spezifische Loci im Genom ausgerichtet ist; oder ein Oligonukleotid, wobei das Oligonukleotid aus DNA, RNA, DNA/RNA-Hybriden und Kombinationen davon ausgewählt ist, umfasst.

4. Verfahren nach Anspruch 1, wobei die DNA-Lokalisierungskomponente ein Protein oder einen Proteinkomplex, das bzw. der dazu fähig ist, ein Merkmal zu erkennen, das aus RNA-DNA-Heteroduplexen, R-Schleifen und Kombinationen davon ausgewählt ist; oder einen Protein oder Proteinkomplex, wobei das Protein oder der Proteinkomplex dazu fähig ist, eine R-Schleife zu erkennen, die aus Cas9, Cascade-Komplex, RecA, RNase H, RNA-Polymerase, DNA-Polymerase und Kombinationen davon ausgewählt ist; oder ein Protein, das dazu fähig ist, eine DNA-Sequenz zu binden, die aus Meganuklease, Zinkfinger-Array, TAL-Array und Kombinationen davon ausgewählt ist, umfasst.

5. Verfahren nach Anspruch 1, wobei die DNA-Lokalisierungskomponente ein Protein, das eine natürlich vorkommende DNA-Bindungsdomäne umfasst; oder ein Protein, das eine natürlich vorkommende DNA-Bindungsdomäne umfasst, wobei die natürlich vorkommende DNA-Bindungsdomäne aus einer bZIP-Domäne, einer Helix-Loop-Helix, einer Helix-Turn-Helix, einer HMG-Box, einem Leucin-Zipper, einem Zinkfinger und Kombinationen davon ausgewählt ist, umfasst.

6. Verfahren nach Anspruch 1, wobei die DNA-Lokalisierungskomponente ein Oligonukleotid, das auf eine Ziellokation in einem Genom ausgerichtet ist; und ein Protein, das dazu fähig ist, an eine Ziel-DNA-Sequenz zu binden, umfasst.

7. Verfahren nach Anspruch 1, wobei die Endonuklease eine Restriktionsendonuklease, Homing-Endonuklease, SI-Nuklease, Mungbohnen-Nuklease, Pankreas-DNAse I, Mikrokokkennuklease, Hefe-HO-Endonuklease oder eine Kombination davon ist.

8. Verfahren nach Anspruch 1, wobei die Endonuklease eine Typ-IIS-Restriktionsendonuklease umfasst.

9. Verfahren nach Anspruch 1, wobei die Endonuklease eine Endonuklease umfasst, die aus der Gruppe bestehend aus AciI, Mn1I, AlwI, BbvI, BccI, BceAI, BsmAI, BsmFI, BspCNI, BsrI, BtsCI, HgaI, HphI, HpyAV, MbolI, MylI, PleI, SfaNI, AcuI, BciVI, BfuAI, BmgBI, BmrI, BpmI, BpuEI, BsaI, BseRI, BsgI, BsmI, BspMI, BsrBI, BsrBI, BsrDI, BtgZI, BtsI, Earl, EciI, MmeI, NmeAIII, BbvCI, Bpu10I, BspQI, SapI, BaeI, BsaXI, CspCI, FokI, BfiI, MboII, Acc36I und Clo051 ausgewählt ist.

10. Verfahren nach Anspruch 1, wobei die Endonuklease BmrI, BfiI, Clo051, FokI oder eine Transposase umfasst.

## Revendications

1. Procédé de modification d'un génome d'un organisme *in vitro* ou ex *vivo* comprenant les étapes consistant à :
a. fournir à une cellule un composant de localisation d'ADN qui est capable de se lier à une séquence d'ADN particulière ; et
b. fournir à la cellule une endonucléase ;
dans lequel ledit composant de localisation d'ADN et ladite endonucléase sont capables de se lier de manière fonctionnelle par l'intermédiaire d'une liaison non covalente,
dans lequel la liaison non covalente comprend un anticorps à domaine unique attaché de manière covalente au composant de localisation d'ADN et dans lequel l'anticorps à domaine unique se lie de manière non covalente à une étiquette d'épitope liée de manière covalente à l'endonucléase à l'intérieur de la cellule ; ou
dans lequel la liaison non covalente comprend un anticorps à domaine unique attaché de manière covalente à l'endonucléase et dans lequel l'anticorps à domaine unique se lie de manière non covalente directement à une étiquette d'épitope liée de manière covalente au composant de localisation d'ADN à l'intérieur de la cellule ;
et dans lequel le génome n'est pas le génome de lignée germinale d'un être humain.

2. Procédé selon la revendication 1 dans lequel ledit composant de localisation d'ADN est choisi parmi un oligonucléotide de liaison à l'ADN, une protéine de liaison à l'ADN, un complexe protéique de liaison à l'ADN et des associations de ceux-ci.

3. Procédé selon la revendication 1 dans lequel ledit composant de localisation d'ADN comprend un oligonucléotide dirigé vers lesdits locus particuliers dans le génome ; ou un oligonucléotide, ledit oligonucléotide étant choisi parmi de l'ADN, de l'ARN, des hybrides ADN/ARN et des associations de ceux-ci.

4. Procédé selon la revendication 1 dans lequel ledit composant de localisation d'ADN comprend une protéine ou un complexe protéique capable de reconnaître un élément caractéristique choisi parmi des hétéroduplex ARN-ADN, des boucles R et des associations de ceux-ci ; ou une protéine ou un complexe protéique, ladite protéine ou ledit complexe protéique étant capable de reconnaître une boucle R choisie parmi Cas9, le complexe Cascade, RecA, la RNase H, l'ARN polymérase, l'ADN polymérase et des associations de ceux-ci ; ou une protéine capable de se lier à une séquence d'ADN choisie parmi une méganucléase, une puce à doigts de zinc, une puce à TAL et des associations de ceux-ci.

5. Procédé selon la revendication 1 dans lequel ledit composant de localisation d'ADN comprend une protéine comprenant un domaine de liaison à l'ADN d'origine naturelle ; ou une protéine comprenant un domaine de liaison à l'ADN d'origine naturelle, ledit domaine de liaison à l'ADN d'origine naturelle étant choisi parmi un domaine bZIP, une hélice-boucle-hélice, une hélice-tour-hélice, une boîte HMG, une glissière à leucine, un doigt de zinc et des associations de ceux-ci.

6. Procédé selon la revendication 1 dans lequel ledit composant de localisation d'ADN comprend un oligonucléotide dirigé vers un endroit cible dans un génome ; et une protéine capable de se lier à une séquence d'ADN cible.

7. Procédé selon la revendication 1 dans lequel ladite endonucléase est une endonucléase de restriction, une endonucléase de homing, la nucléase SI, la nucléase du haricot mungo, la DNase pancréatique I, la nucléase micrococcale, l'endonucléase HO de levure ou une association de celles-ci.

8. Procédé selon la revendication 1 dans lequel ladite endonucléase comprend une endonucléase de restriction de type IIS.

9. Procédé selon la revendication 1 dans lequel ladite endonucléase comprend une endonucléase choisie dans le groupe constitué par AciI, Mn1I, AlwI, BbvI, BccI, BceAI, BsmAI, BsmFI, BspCNI, BsrI, BtsCI, HgaI, HphI, HpyAV, Mbo1I, My1I, PleI, SfaNI, AcuI, BciVI, BfuAI, BmgBI, BmrI, BpmI, BpuEI, BsaI, BseRI, BsgI, BsmI, BspMI, BsrBI, BsrBI, BsrDI, BtgZI, BtsI, EarI, EciI, MmeI, NmeAIII, BbvCI, Bpu10I, BspQI, SapI, BaeI, BsaXI, CspCI, FokI, BfiI, MboII, Acc36I et Clo051.

10. Procédé selon la revendication 1 dans lequel ladite endonucléase comprend BmrI, BfiI, Clo051, FokI ou une transposase.


### SEQUENCE LISTING

<110> Poseida Therapeutics, Inc. Ostertag, Eric M Yeshi, Tseten Li, Xianghong
<120> METHOD OF DIRECTING PROTEINS TO SPECIFIC LOCI IN THE GENOME AND USES THEREOF
<130> POTH-001/001WO
<150> 62/013,382
   <151> 2014-06-17
<150> 62/163,565
   <151> 2015-05-19
<160> 3
<170> PatentIn version 3.5
<210> 1
   <211> 18
   <212> DNA
   <213> Artificial Sequence
<220>
   <223> Synthetic oligonucleotide
<400> 1
   ggggccggag ccgcagtg 18
<210> 2
   <211> 18
   <212> PRT
   <213> Artificial Sequence
<220>
   <223> Synthetic oligopeptide
<400> 2
   Ser Ser Gly Gly Gly Gly Ser Gly Gly Gly Gly Gly Gly Ser Ser Arg
   1               5                   10                  15
   Ser Ser
<210> 3
   <211> 7
   <212> PRT
   <213> Artificial Sequence
<220>
   <223> Synthetic oligopeptide
<400> 3
   Gly Gly Ser Ser Arg Ser Ser
   1               5